# FASCICULE DE BREVET EUROPEEN

(11) **EP 3 016 079 B1**
(45) Date de publication et mention de la délivrance du brevet: **17.05.2017**
(21) Numéro de dépôt: 15192284.6
(22) Date de dépôt: 30.10.2015
(51) Int. Cl.: G07F 7/00, H05K 5/00

(54) **BOITIER DE TERMINAL ÉQUIPÉ D'UNE TRAPPE AMOVIBLE À PROFIL CURVILIGNE**
GEHÄUSE EINES ENDGERÄTS, DAS MIT EINER ABNEHMBAREN KLAPPE MIT GEBOGENEM PROFIL AUSGERÜSTET IST
TERMINAL HOUSING PROVIDED WITH A REMOVABLE HATCH WITH A CURVED PROFILE

(30) Priorité: 03.11.2014 FR 1460553
(43) Date de publication de la demande: 04.05.2016
(73) Titulaire: Ingenico Group, 75015 Paris (FR)
(72) Inventeur: BONNET, Eric, 26120 Malissard (FR); YERNAUX, Olivier, 26100 Romans-Sur-Isere (FR)
(74) Mandataire: Vidon Brevets & Stratégie

(56) Documents cités:
- EP-A1- 1 128 347
- EP-A1- 2 101 280
- WO-A1-2011/080117

## Description

### 1. DOMAINE DE L'INVENTION

Le domaine de l'invention est celui des boitiers de terminaux, et plus particulièrement des boitiers de terminaux de paiement électroniques.

L'invention porte plus précisément sur un boitier de terminal équipé d'une trappe amovible présentant un profil curviligne.

### 2. ARRIÈRE-PLAN TECHNOLOGIQUE

On s'attache plus particulièrement dans la suite de ce document à décrire la problématique existant dans le domaine des boitiers de paiement électroniques, à laquelle ont été confrontés les inventeurs de la présente demande de brevet. L'invention ne se limite bien sûr pas à ce domaine particulier d'application, mais présente un intérêt pour tout boitier de terminal de communication devant faire face à une problématique proche ou similaire.

Les boitiers des terminaux de paiement électroniques actuels présentent en général deux parties, notées capot inférieur et capot supérieur, permettant d'abriter tous les composants et éléments de fonctionnement du terminal (composants électroniques, rouleau papier pour impression, etc.).

Le capot inférieur comprend un logement pouvant être refermé par l'intermédiaire d'une trappe de protection amovible. Un tel logement permet de loger des éléments du terminal, tel que par exemple une batterie, une carte à puce, des connexions pour câbles de liaison du terminal ou un module de mémorisation transactionnel. Ce dernier, en particulier, est dédié à l'enregistrement des transactions effectuées par le terminal de façon à y inclure des fonctionnalités de caisse enregistreuse, de journalisation de transactions, etc.

Traditionnellement, la trappe de protection est fixée au capot inférieur au moyen d'une pluralité de vis de fixation ou d'un mécanisme d'emmanchage à force.

L'une des préoccupations principales des constructeurs est de concevoir et de fabriquer des terminaux de paiement électroniques qui soient peu encombrants, de bonne tenue mécanique et d'une grande simplicité d'utilisation.

Or un des inconvénients liés à la présence de vis de fixation réside dans le manque de place pour placer ces vis et l'augmentation de l'épaisseur du terminal. En effet, les boitiers actuels doivent s'adapter à la diminution de la taille des terminaux demandée par les utilisateurs, imposant notamment des contraintes dimensionnelles et ergonomiques.

Un autre inconvénient lié à l'utilisation de vis de fixation est de rendre les opérations de montage et de démontage de la trappe relativement longues et fastidieuses, de part la nécessité de faire appel à un outil adapté (tournevis par exemple).

Quant au mécanisme d'emmanchage à force, ils présentent les inconvénients d'être fragiles à l'usage, et de rendre les opérations d'ouverture et de fermeture de la trappe malaisées et fastidieuses.

Les constructeurs ont également mis au point un système de montage basé sur un coulissement de la trappe sur le capot inférieur au moyen de rails de guidage. Ces rails de guidage permettent un mouvement de translation rectiligne de la trappe par rapport au capot inférieur, dans un premier sens, pour procéder au montage (ou à la fermeture) de la trappe ou, dans un deuxième sens, pour procéder au démontage (ou à l'ouverture) de la trappe.

Toutefois, ce type de système n'est compatible qu'avec des trappes présentant un profil de surface rectiligne. Or la plupart des boitiers conçus à heure actuelle présentent un profil de surface curviligne, notamment pour répondre à des contraintes d'ergonomie, nécessitant la mise en place d'une trappe à profil de surface curviligne.

### 3. OBJECTIFS DE L'INVENTION

L'invention, dans au moins un mode de réalisation, a notamment pour objectif de pallier ces différents inconvénients de l'état de la technique.

Plus précisément, dans au moins un mode de réalisation de l'invention, un objectif est de fournir un boitier de terminal permettant un montage et un démontage aisés d'une trappe à profil de surface curviligne, tout en tenant compte des contraintes dimensionnelles et ergonomiques.

Au moins un mode de réalisation de l'invention a également pour objectif de fournir un boitier de terminal qui tient compte des contraintes liées à l'épaisseur et au profil curviligne du boitier.

Au moins un mode de réalisation de l'invention a également pour objectif de fournir un boitier de terminal qui présente une bonne tenue mécanique, notamment un boitier qui soit résistant aux chutes.

### 4. EXPOSÉ DE L'INVENTION

Dans un mode de réalisation particulier de l'invention, il est proposé un boitier de terminal comprenant au moins un capot supérieur et un capot inférieur dans lequel est ménagé un logement pouvant être refermé au moyen d'une trappe amovible présentant un profil curviligne. La trappe comprend une pluralité de rainures réparties longitudinalement sur au moins deux faces parallèles de la trappe en suivant ledit profil curviligne, le capot inférieur comprend une pluralité d'ergots répartis longitudinalement sur au moins deux faces parallèles du capot inférieur en suivant ledit profil curviligne, chaque ergot étant destiné à coopérer avec une rainure associée sur la trappe. Les ergots et les rainures sont conformés de sorte à permettre :
- au moyen d'une première combinaison de mouvements comprenant un mouvement de translation suivi d'un mouvement de rotation de la trappe, une insertion et un guidage respectivement des ergots dans les rainures de la trappe, lorsque la trappe est insérée sur le capot inférieur,
- au moyen une deuxième combinaison de mouvements comprenant un mouvement de rotation suivi d'un mouvement de translation de la trappe, un guidage des ergots dans les rainures de la trappe et un retrait des ergots des rainures respectivement, lorsque la trappe est retirée du capot

Ainsi, l'invention repose sur une nouvelle mise en oeuvre d'un boitier de terminal permettant un montage et démontage d'une trappe amovible à profil curviligne et du capot inférieur de manière aisée.

Ainsi, l'invention, dans ses différents modes de réalisation particuliers, permet d'optimiser les opérations d'assemblage et de démontage de la trappe amovible par rapport au capot inférieur du boitier en utilisant des rainures et des ergots astucieusement répartis sur la trappe et le capot inférieur respectivement, et coopérant ensemble de façon à autoriser une insertion et un guidage des ergots dans les rainures lorsque la trappe est insérée sur le capot selon une première combinaison de mouvements, et un retrait et un guidage des ergots dans les rainures lorsque la trappe retirée du capot, selon une deuxième combinaison de mouvements de la trappe par rapport au capot. De telles combinaisons de mouvements à base de translation et de rotation sont naturelles pour l'utilisateur et facilite grandement la manipulation de la trappe à profil curviligne, par exemple lors de la maintenance du boitier.

De plus, les ergots et rainures selon l'invention permettent de s'affranchir de l'utilisation de vis de fixation, qui rendraient les opérations d'ouverture et de fermeture de la trappe fastidieuses, tout en assurant une fermeture efficace de la trappe sur le capot. En effet, ces ergots et rainures étant réalisés dans un matériau élastiquement déformable, présentent une certaine souplesse permettant de ne pas casser et donc permettant à la trappe de ne pas s'ouvrir inopinément lors d'une chute par exemple, contrairement aux vis de fixation qui se détériorent lors d'une chute (parce que les parties autour des vis se cassent ou fêlent. Par ailleurs, ces ergots et rainures sont plus faciles à mettre au point, lors des phases de conception du boitier, que des fixations par vis, les pièces à mettre au point pouvant être réalisées par moulage à partir d'une matière élastiquement déformable (un polymère par exemple), alors que les vis, éléments du commerce, sont plus difficiles à modifier.

Selon un aspect particulier de l'invention, la trappe présente un sens d'insertion et un sens de retrait opposé au sens d'insertion. Pour chaque face de la trappe, les rainures sont chacune inclinée par rapport audit profil curviligne d'un angle d'inclinaison qui est croissant dans le sens de retrait de la trappe et décroissant dans le sens d'insertion de la trappe. Pour chaque face du capot, les ergots sont chacun incliné par rapport audit profil curviligne d'un angle d'inclinaison qui est croissant dans le sens de retrait de la trappe et décroissant dans le sens d'insertion de la trappe.

Autrement dit, le mouvement de rotation de la trappe par rapport au capot inférieur se fait autour d'un centre de rotation commun pour tous les ergots (ou toutes les rainures) mais suivant des rayons de courbure différents selon la position de l'ergot (ou de la rainure) : le rayon de courbure est croissant dans le sens de retrait de la trappe et décroissant dans le sens d'insertion de la trappe.

De cette manière, les forces mises en jeu lors de la mise en place de la trappe sur le capot croissent au fur et à mesure que l'utilisateur insère la trappe sur le capot, assurant une bonne stabilité de la trappe en fin de course. Inversement, les forces mises en jeu lors du retrait de la trappe décroissent rapidement, assurant ainsi un retrait aisé et naturel de la trappe.

Selon une caractéristique particulière, chaque rainure de la pluralité comprend :
- une portion d'insertion permettant une insertion en translation de l'ergot, associée à ladite rainure, dans ladite rainure, et
- une portion de guidage permettant un guidage en rotation dudit ergot dans ladite rainure.

Ainsi, les rainures prévues dans la trappe comprennent chacune une portion d'insertion configurée pour permettre un accostage en translation de la trappe dans un premier temps et une portion de guidage configurée pour permettre un guidage en rotation de la trappe dans un second temps, lors de son report sur le capot inférieur, et vice versa.

Selon une caractéristique particulière, chaque rainure se présente sensiblement sous la forme d'un L allongé longitudinalement, avec une partie latérale et une partie longitudinale, ladite partie latérale formant la portion d'insertion et ladite partie longitudinale formant la portion de guidage.

Selon une caractéristique particulière, chaque ergot se présente sous la forme d'un parallélépipède.

Selon un aspect particulier de l'invention, les rainures sont placées sur des faces latérales de la trappe, et les ergots sont placés sur des faces latérales du capot.

Selon un aspect particulier de l'invention, les rainures sont réparties avec des espacements homogènes (équidistants par exemple) sur les faces latérales de la trappe et les ergots sont répartis avec des espacements homogènes (équidistants par exemple) sur les faces latérales du capot, correspondant aux espacements des rainures de la trappe.

Une telle configuration permet une répartition équilibrée des forces exercées au niveau des rainures et des ergots lors de chutes du boitier par exemple. Elle permet également de conformer la trappe sur le capot (pour rattraper d'éventuelles déformations de la trappe dues au moulage).

Selon une caractéristique particulière, le boitier de terminal comprend des moyens de verrouillage réversibles de la trappe au capot inférieur, constitué d'au moins une patte d'accrochage élastiquement flexible et orientée longitudinalement sur une extrémité avant de la trappe, coopérant avec au moins une encoche de réception de ladite au moins une patte d'accrochage, ménagée dans le capot inférieur.

Ainsi, les moyens de verrouillage selon l'invention sont configurés de sorte à permettre une retenue élastique de la trappe au capot inférieur, mais qui sont libérables lorsque une force de compression perpendiculaire au profil curviligne est exercée à proximité de ladite extrémité avant de la trappe, de sorte que la trappe passe d'une position de verrouillage à une position de déverrouillage.

Grâce à ces moyens de verrouillage réversibles, l'invention prévoit la possibilité de réaliser :
- un « verrouillage » de la trappe en fin de course, par simple « clipsage » de la patte d'accrochage dans l'encoche de réception correspondante, ce qui permet de rendre solidaire la trappe et le capot inférieur lorsque la trappe est reportée sur le capot, et
- un « déverrouillage » de la trappe par simple effort de pression sur la trappe pour désengager la patte d'accrochage de l'encoche de réception correspondante, autorisant ainsi le retrait de la trappe du boitier.

Selon une variante de réalisation, la trappe comprend un bouton poussoir mobile en translation, coopérant avec au moins une patte d'accrochage mobile en translation, ladite au moins une patte d'accrochage étant orientée longitudinalement sur une extrémité avant de la trappe, coopérant avec au moins une encoche de réception de ladite au moins une patte d'accrochage, ménagée dans le capot inférieur.

Cette variante permet un montage ou un démontage simple et très pratique pour l'opérateur.

Selon une caractéristique particulière, la trappe comprend une portion en forme de anse au niveau de son extrémité arrière conformée de façon à assurer un verrouillage d'au moins un connecteur situé à l'arrière du capot inférieur.

Le connecteur est alors pris en sandwich entre la portion en forme de anse de la trappe et l'arrière du capot inférieur, le rendant ainsi solidaire de la trappe et du capot.

### 5. LISTE DES FIGURES

D'autres caractéristiques et avantages de l'invention apparaîtront à la lecture de la description suivante, donnée à titre d'exemple indicatif et non limitatif, et des dessins annexés, dans lesquels :
- les figures 1a et 1b présentent chacune une vue en perspective de dessous d'un boitier de terminal de paiement selon un mode de réalisation particulier de l'invention, la figure 1a illustrant le boitier avec la présence d'une trappe et la figure 1b illustrant le boitier en l'absence de trappe ;
- les figures 2a, 2b, 3a et 3b illustrent, de façon schématique, le principe de montage et de démontage d'une trappe amovible sur le capot inférieur d'un boitier de terminal selon un mode de réalisation particulier de l'invention;
- les figures 3c et 3d présentent la trappe amovible respectivement en position « engagée » sur le capot inférieur et en position fermée selon un mode de réalisation de l'invention ;
- la figure 4 présente une vue détaillée en perspective d'une extrémité du capot inférieur après report de la trappe sur celui-ci ;
- la figure 5 présente une vue en perspective de dessous de la trappe illustrée aux figures 2a, 2b, 3a et 3b ;
- la figure 6 présente une vue en coupe perspective partielle de la trappe une fois montée sur le capot inférieur du boitier, illustrant la présence d'un connecteur du terminal pris en sandwich entre la trappe et le capot inférieur du boitier.

### 6. DESCRIPTION DÉTAILLÉE

Sur toutes les figures du présent document, les éléments et étapes identiques sont désignés par une même référence numérique.

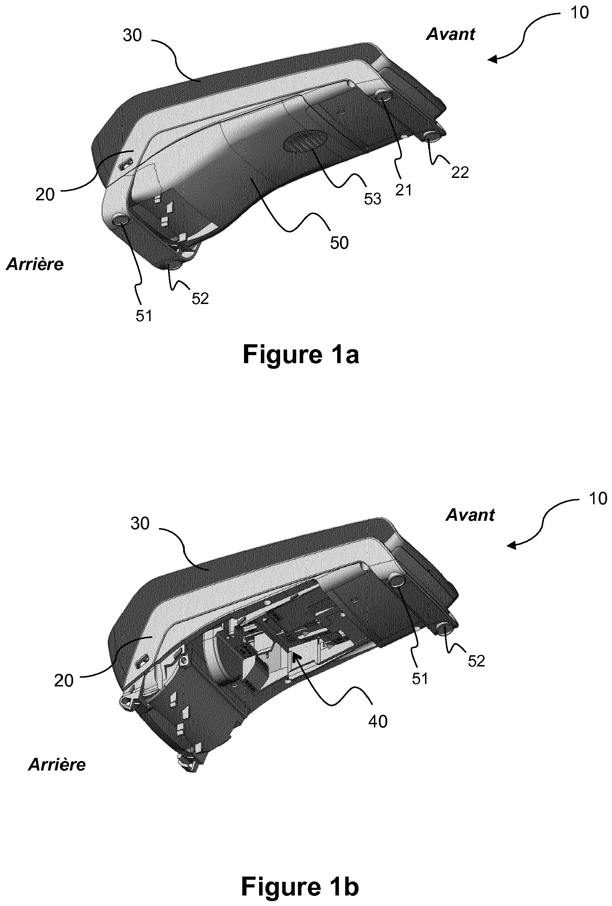

Selon un mode de réalisation particulier de l'invention, illustré par les **figures 1a** et **1b****,** le boitier 10 de terminal de paiement est constitué d'un capot supérieur 30 et un capot inférieur 20 fixés l'un à l'autre par des vis de fixation ou des moyens de fixation articulée par exemple.

Le capot inférieur 20 comprend un logement 40 qui peut être refermé par l'intermédiaire d'une trappe de protection amovible, référencée 50.

Le logement 40 est conçu pour pouvoir recevoir des éléments du terminal, tel que par exemple une batterie, une carte à puce, un module électronique et/ou des câbles de connexion.

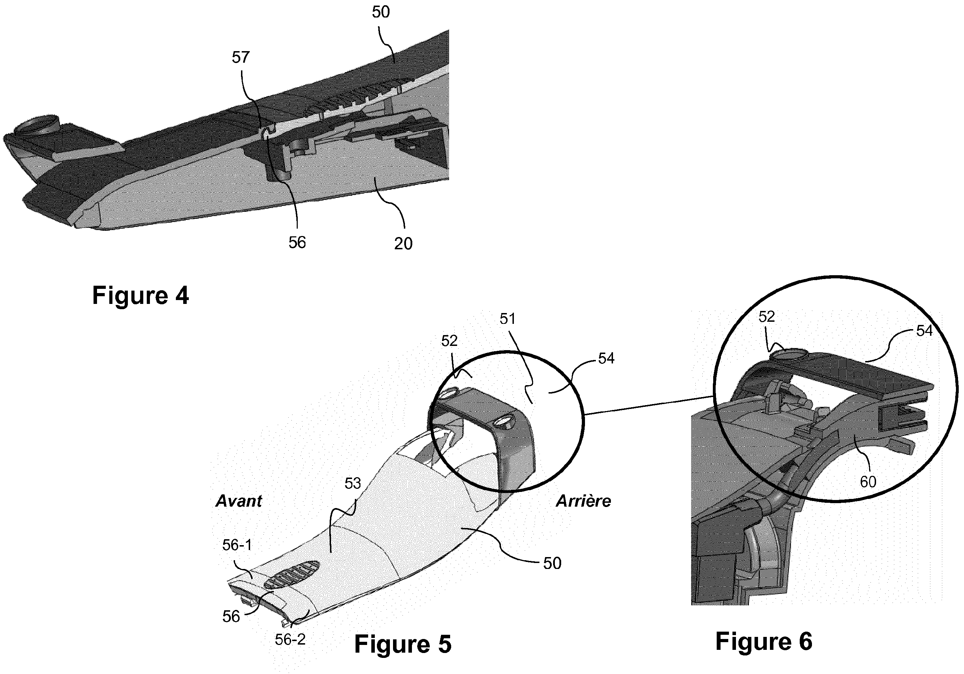

Du fait du profil globalement curviligne du capot inférieur 20 sur lequel la trappe 50 est destinée à être montée, la trappe 50 présente un profil de surface curviligne (on parle aussi de profil à plan curviligne). Ceci est bien illustré à la figure 4.

Le boitier 10 présente en outre une fente d'insertion de carte (non représentée) à l'avant du boitier et une fente de sortie de papier d'impression (non représentée) à l'arrière du boitier.

Deux plots en caoutchouc 21, 22 sont en outre prévus sur la face de dessous du capot inférieur 20 à l'avant du boitier 10 et deux plots en caoutchouc 51, 52 sur la face de dessous de la trappe 50 à l'arrière du boitier 10. Ces quatre plots en caoutchouc 21, 22, 51, 52 assurent le maintien et la stabilité du boitier 10 lorsque celui-ci est placé sur un support, tel qu'une table ou un comptoir par exemple. Bien sûr, il s'agit d'un exemple de réalisation et il est possible d'envisager d'autres variantes assurant la même fonction. Par exemple, on pourrait envisager de remplacer les deux plots 21, 22 par un unique plot en caoutchouc de plus grande dimension.

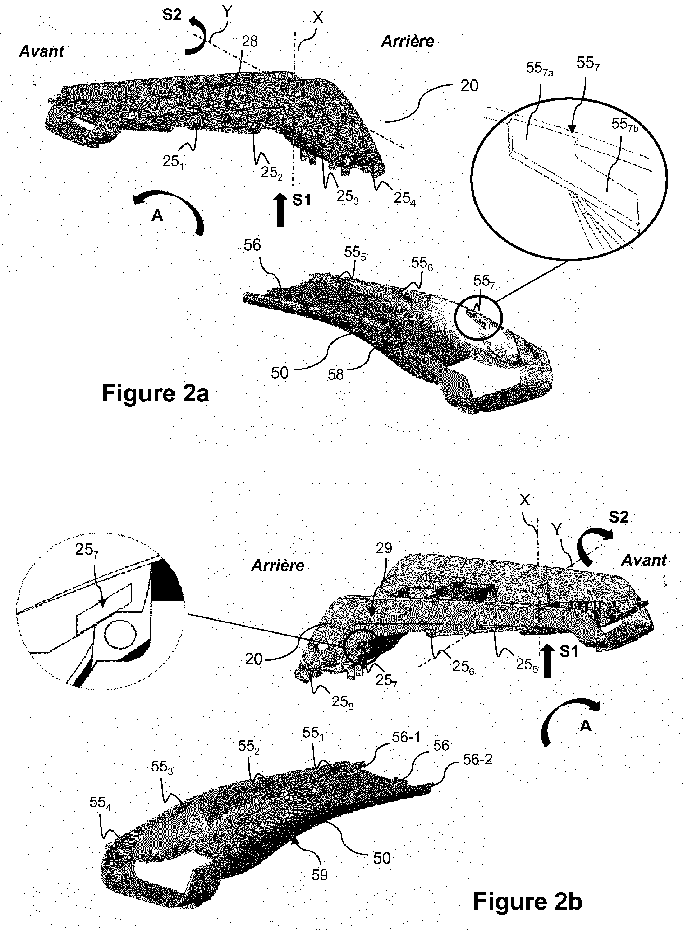

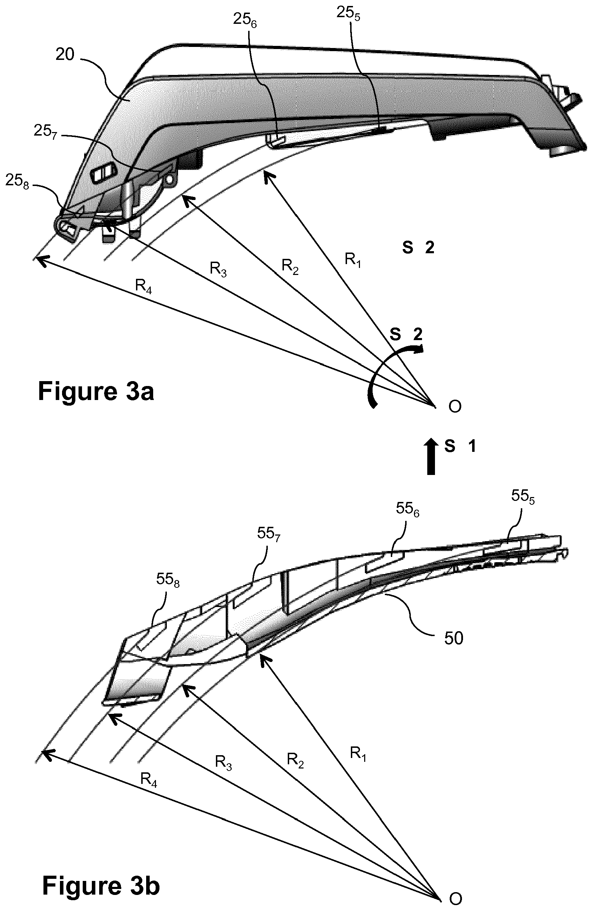

Les **figures 2a, 2b****,** **3a et 3b** illustrent de façon schématique le principe de montage et de démontage de la trappe 50 et du capot inférieur 20 d'un boitier de terminal, selon un mode de réalisation particulier de l'invention. La figure 2a représente une première vue de côté et la figure 2b représente une seconde vue de côté de la trappe 50 et du capot inférieur 20.

La trappe 50 comprend un ensemble de huit de rainures latérales 55₁ à 55₈ réparties longitudinalement sur les faces latérales 58 et 59 de ladite trappe en suivant le profil de surface curviligne. La face latérale 58 comprend les quatre rainures 55₁ à 55₄ et la face latérale 59 comprend les quatre rainures 55₅ à 55₈.

Le capot inférieur 20 comprend un ensemble de huit ergots latéraux 25₁ à 25₈ répartis longitudinalement sur les faces latérales 28 et 29 dudit capot en suivant le profil de surface curviligne. La face latérale 28 comprend les quatre ergots référencés 25₁ à 25₄ et la face latérale 29 comprend les quatre ergots référencés 25₅ à 25₈. Les ergots 25₁ à 25₈ du capot sont destinés à coopérer avec les rainures 55₁ à 55₈ de la trappe respectivement. L'ensemble des huit de rainures latérales 55₁ à 55₈ et l'ensemble des huit ergots latéraux 25₁ à 25₈ forment plus généralement des moyens de guidage de la trappe sur le capot inférieur.

La flèche symbolisée par la lettre « A » représente le sens d'insertion global de la trappe 50 sur le capot inférieur 20. Le sens opposé à ce sens d'insertion correspond au sens de retrait de la trappe 50 du capot inférieur 20.

Chaque rainure ménagée dans la trappe 50 se présente sensiblement sous la forme d'un L allongé longitudinalement dans le sens de retrait de la trappe, avec une partie latérale et une partie longitudinale. Prenons par exemple la rainure 55₇, illustrée en gros plan sur la figure 2a. La partie latérale à de la rainure 55₇, située au bord de la face latérale (côté report de la trappe), forme une portion d'insertion permettant une insertion de l'ergot 25₇ qui lui est associé, dans la rainure 55₇. La partie longitudinale 55_{7b} forme une portion de guidage permettant un guidage de l'ergot 25₇ dans la rainure 55₇, lorsque la trappe 50 est insérée sur le capot inférieur 20, jusqu'à une position de fin de course.

Sur la face latérale 58 de la trappe, les rainures 55₁ à 55₄ sont inclinées d'un angle d'inclinaison qui est décroissant dans le sens d'insertion (flèche A) de la trappe 50 (i.e. depuis l'arrière vers l'avant du capot), et inversement qui est croissant dans le sens de retrait de la trappe 50 (i.e. depuis l'avant vers l'arrière du capot). Sur la face latérale 59 de la trappe, les rainures 55₅ à 55₈ sont inclinées d'un angle d'inclinaison qui est décroissant dans le sens d'insertion (flèche A) de la trappe 50 (i.e. depuis l'arrière vers l'avant du capot), et inversement qui est croissant dans le sens de retrait de la trappe 50 (i.e. depuis l'avant vers l'arrière du capot).

Sur la face latérale 28 du capot, les ergots 25₁ à 25₄ présentent un angle d'inclinaison décroissant identique aux rainures 55₁ à 55₄ associées sur la trappe. Sur la face latérale 29 de la trappe, les ergots 25₅ à 25₈ présentent un angle d'inclinaison décroissant identique aux rainures 55₅ à 55₈ associées sur la trappe.

Les ergots 25₁ - 25₈ dans ce mode de réalisation se présentent chacun sous la forme d'un parallélépipède. Cette forme est particulièrement bien adaptée pour faciliter l'insertion et le guidage des ergots dans les rainures correspondantes en forme de L allongé.

### ➢ Montage de la trappe et du capot inférieur

La trappe 50 est reportée sur le capot inférieur 20 à l'aide d'une combinaison de mouvements comprenant un mouvement de translation et un mouvement de rotation.

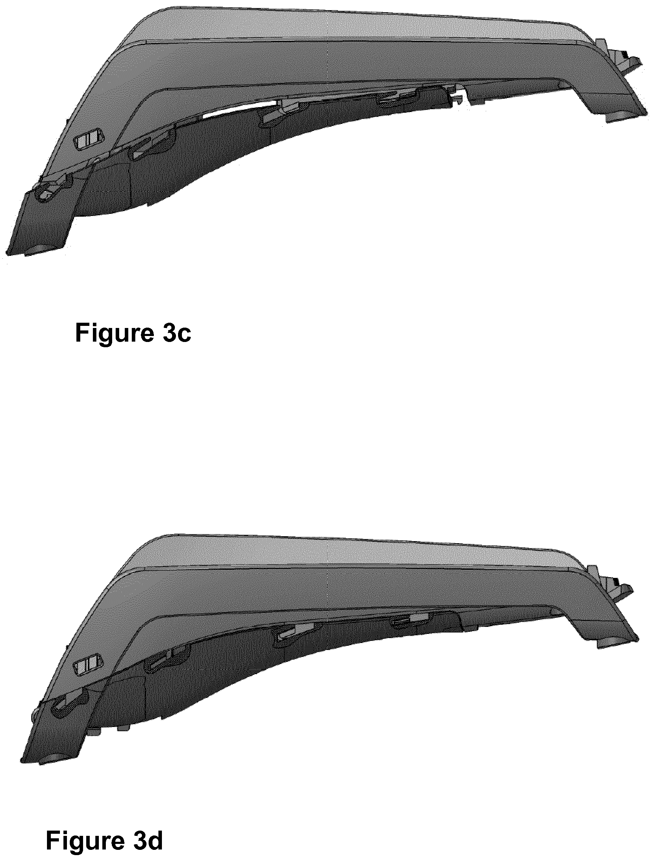

Comme illustré sur les figures 3a et 3b, la trappe 50 est insérée, dans un premier temps, sur le capot inférieur 20 à l'aide d'un mouvement de translation de la trappe par rapport au capot de telle sorte que les ergots (25₅ à 25₈) du capot viennent s'insérer dans les portions d'insertion (55₅ₐ à 55₈ₐ) ménagées dans la trappe. Ce mouvement de translation permet de réaliser un « accostage » de la trappe 50 sur le capot 20. Comme illustré en figure 3c, la trappe se trouve donc dans une position « engagée » sur le capot inférieur, après l'accostage.

Dans un deuxième temps, la trappe 50 est guidée dans le capot à l'aide d'un mouvement de rotation de la trappe par rapport au capot de telle sorte que les ergots (25₅ à 25₈) sont guidés en rotation dans les portions de guidage (55_{5b} à 55_{8b}) correspondantes ménagées dans la trappe 50, jusqu'à ce que la trappe se retrouve dans une position de verrouillage, comme illustré en figure 3d (le principe de verrouillage de la trappe en fin de course est détaillé plus loin en relation avec la figure 4).

L'angle d'inclinaison des ergots et des rainures est choisi de façon à permettre une insertion de la trappe 50 sur le capot inférieur 20 au moyen d'une rotation autour d'un axe virtuel de rotation commun O selon des rayons de courbure différents en fonction du couple ergot/rainure concerné. En particulier, les ergots et rainures présentent des rayons de courbure décroissants dans le sens d'insertion de la trappe. Les rayons R₁, R₂, R₃, R₄ sont associés respectivement aux couples 25₅/50₅, 25₆/50₆, 25_{7/}50₇, 25₈/50₈, R₁ étant le rayon de courbure le plus faible et R₄ le rayon de courbure le plus fort.

Ainsi, du fait de l'agencement et de la forme des ergots et rainures, le report de la trappe 50 sur le capot inférieur 20 est réalisé au moyen d'un mouvement de translation selon l'axe vertical X (dans le sens de la flèche S1), d'une part, autorisant un accostage de la trappe 50 sur le capot inférieur 20, et d'autre part, un mouvement de rotation autour de l'axe transverse du capot Y (dans le sens de la flèche S2), autorisant un guidage de la trappe 50 dans le capot inférieur 20. En d'autres termes, l'axe d'insertion de la trappe 50 sur le capot inférieur 20 est modifié au cours de son insertion, pour permettre de respecter le profil curviligne de la trappe 50.

### ➢ Démontage de la trappe et du capot inférieur

Le démontage de la trappe 50 du capot inférieur 20 est effectué à l'aide d'une combinaison de mouvements inverse de celle utilisée pour le montage, comprenant un mouvement de rotation suivi d'un mouvement de translation.

Après déverrouillage de la trappe 50 (le principe de déverrouillage est détaillé plus loin en relation avec la figure 4), la trappe 50 est guidée dans le capot inférieur à l'aide d'un mouvement de rotation de la trappe par rapport au capot de telle sorte que les ergots (25₅ à 25₈) sont guidés en rotation dans les portions de guidage (55_{5b} à 55_{8b}) de la trappe 50. L'angle d'inclinaison des ergots et des rainures est choisi de façon à permettre un guidage de la trappe 50 au moyen d'une rotation autour d'un axe virtuel de rotation commun O selon les rayons de courbure R₁, R₂, R₃, R₄ associés respectivement aux couples 25₅/50₅, 25₆/50₆, 25₇/50₇, 25₈/50₈. Le mouvement en rotation se fait en sens inverse du sens de la flèche S2). Une fois les ergots (25₅ à 25₈) placés dans les portions d'insertion correspondantes (55₅ₐ à 55₈ₐ), un mouvement de translation de la trappe par rapport au capot dans le sens inverse du sens de la flèche S1 est réalisé, de telle sorte que les ergots (25₅ à 25₈) du capot se désengagent des portions d'insertion (55₅ₐ à 55₈ₐ) ménagées dans la trappe, de sorte que la trappe soit retirée du capot. Ainsi, l'axe de retrait de la trappe 50 du capot inférieur 20 est modifié au cours de son retrait, pour permettre de respecter le profil curviligne de la trappe 50.

Une telle combinaison de mouvements de translation et rotation, que ce soit pour le montage de la trappe 50 (insertion au moyen d'une translation puis d'une rotation) ou son démontage (retrait au moyen d'une rotation puis d'une translation), est naturel pour l'opérateur et facilite grandement la manipulation de la trappe à profil curviligne et du capot, par exemple lors de la maintenance du boitier 10 (changement d'éléments compris dans le logement 40 du capot inférieur 20 du terminal (batterie, carte à puce, module électronique, câbles de connexion, etc.).

Il est à noter que le boitier de terminal selon l'invention ne nécessite pas d'outil ou de matériel supplémentaire pour réaliser les opérations d'insertion et de retrait, contrairement à l'utilisation de vis de fixation. En effet, les ergots et rainures selon l'invention permettent de s'affranchir de l'utilisation de vis de fixation, qui rendraient les opérations d'ouverture et de fermeture de la trappe fastidieuses, tout en assurant une fermeture efficace de la trappe sur le capot (notamment grâce à l'inclinaison décroissante des rainures dans le sens d'insertion de la trappe). De plus, ces ergots et rainures selon l'invention peuvent être réalisés dans un matériau élastiquement déformable, présentant une certaines souplesse et permettant ainsi de ne pas casser. Par ailleurs, ces ergots et rainures sont faciles à mettre au point, lors des phases de conception du boitier, les pièces à mettre au point pouvant être réalisées par moulage à partir d'une matière élastiquement déformable (un polymère par exemple), alors que les vis, éléments du commerce, sont plus difficiles à modifier.

Il est à noter également que les rainures 25₁ - 25₈ ménagées dans la trappe 50 sont réparties sur les faces latérales 28 et 29 avec des espacements homogènes, comme pour les ergots 55₁ - 55₈ du capot inférieur qui sont répartis sur les faces latérales avec des espacements équidistants correspondants. Ceci permet de répartir les efforts sur les pièces et ainsi éviter une déformation voire même une casse de pièces. Bien entendu, un agencement différent des ergots et rainures pourrait être envisagé, sans sortir du cadre de l'invention.

Dans le mode de réalisation représenté sur ces figures, la trappe contient un ensemble de huit rainures (soit quatre rainures par face) et le capot inférieur contient un ensemble de huit ergots (soit quatre ergots par face). Il s'agit ici d'un exemple illustratif et on peut prévoir un nombre d'ergots et de rainures plus ou moins important, sans sortir du cadre de l'invention. Un nombre plus important d'ergots et de rainures peut être envisagé par exemple si l'on souhaite renforcer la résistance aux chutes du boitier ou bien en fonction du design du boitier tout simplement.

Le boitier de terminal 10 comprend en outre des moyens de verrouillage réversibles de la trappe 50 au capot inférieur 20. Dans l'exemple illustré sur les **figures 4** et **5****,** les moyens de verrouillage sont constitués d'une patte d'accrochage 56 et de deux pattes de guidage 56-1, 56-2.

La patte d'accrochage 56 est élastiquement flexible et orientée longitudinalement sur l'extrémité libre avant de la trappe 50, coopérant avec une encoche de réception 57 ménagées dans le capot inférieur 20 du boitier. La patte d'accrochage 56 et l'encoche de réception 57 correspondante sont configurées de sorte à permettre une retenue élastique de la trappe au capot inférieur, conférant à la trappe une position de verrouillage (en fin de course d'enfoncement des ergots dans les portions de guidage correspondantes), libérable lorsque une force de pression est exercée sensiblement perpendiculairement à la surface de la trappe et à proximité de l'extrémité avant de celle-ci, de telle sorte que la trappe passe de la position de verrouillage à une position de déverrouillage. La zone de pression 53 par exemple peut être dédiée à cette fonction de verrouillage/déverrouillage de la trappe 50.

Les pattes de guidage 56-1 et 56-2 assurent le guidage en fin de course de la trappe 50 en coopérant chacune avec une encoche de réception associée.

Ainsi, ces moyens de verrouillage assurent un « verrouillage » de la trappe 50 en fin de course lors de son insertion dans le capot 20, par simple « clipsage » de la patte d'accrochage 56 dans l'encoche 57, rendant la trappe 50 solidaire du capot inférieur 20 lorsque la trappe 50 est reportée sur le capot 20. Ces moyens de verrouillage étant réversibles, ils permettent en outre un « déverrouillage » de la trappe 50 en réalisant une simple pression sur la zone de pression 53 de manière à désengager la patte d'accrochage 56 de son encoche de réception 57, autorisant ainsi le retrait de la trappe 50 du capot inférieur 20.

Les moyens de verrouillage réversibles sont ainsi conçus pour permettre un verrouillage et un déverrouillage de la trappe au moyen de la pression d'un seul doigt (sur la zone de pression dédiée 53). Ainsi, la trappe peut être mise en place et retirée de façon ergonomique.

Selon une variante de réalisation, la zone de pression peut être substituée par un bouton pressoir mobile en translation et coopérant avec la patte d'accrochage 56 également mobile en translation, pour permettre de verrouiller et de déverrouiller la trappe 50 sur le terminal 10, par une simple pression manuelle sur le bouton.

De manière avantageuse, la trappe 50 comprend en outre une portion en forme de anse 54 au niveau de son extrémité arrière. Comme illustré en gros plan sur la **figure 6****,** cette portion en forme de anse 54 est conformée de façon à assurer un verrouillage ou un blocage d'au moins un connecteur 60 situé à l'arrière du capot inférieur. Ainsi, une fois la trappe 50 insérée et verrouillée sur le capot inférieur 20, le connecteur 60 est pris en sandwich entre l'anse 54 de la trappe et l'arrière du capot inférieur 20, le rendant solidaire de la trappe et du capot.

Le mode de réalisation décrit ci-dessus s'applique aux boitiers de paiement électroniques. Il est clair toutefois que l'invention ne se limite pas à cette application particulière et peut aisément être adapté à d'autres types de boitier de terminal de communication, sans sortir du cadre de l'invention.

L'agencement des rainures et des ergots illustrés dans le cadre du mode de réalisation décrit ci-dessus en relation avec les figures 1 à 6 est tel que les rainures et ergots sont distribués respectivement sur les faces latérales de la trappe et le capot inférieur. Bien entendu, il ne s'agit que d'un exemple de réalisation particulier. On pourrait envisager, à titre d'alternative, d'avoir les rainures et ergots distribués respectivement sur les faces latérales du capot inférieur et de la trappe, sans sortir du cadre de la présente invention (le principe décrit ci-dessus en relation avec les figures 1 à 6 s'appliquant de la même manière, seul le type des éléments de guidage (ergots ou rainures) étant interverti).

## Revendications

1. Boitier de terminal (10) comprenant au moins un capot supérieur (30) et un capot inférieur (20) dans lequel est ménagé un logement (40) pouvant être refermé au moyen d'une trappe amovible (50) présentant un profil curviligne, **caractérisé en ce que** :
- la trappe, ou le capot inférieur, comprend une pluralité de rainures (55₁-55₈) réparties longitudinalement sur au moins deux faces parallèles en suivant ledit profil curviligne,
- le capot inférieur, ou la trappe respectivement, comprend une pluralité d'ergots (25₁-25₈) répartis longitudinalement sur au moins deux faces parallèles en suivant ledit profil curviligne, chaque ergot étant destiné à coopérer avec une rainure associée,
**en ce que** les ergots et les rainures sont conformés de sorte à permettre :
- au moyen d'une première combinaison de mouvements comprenant un mouvement de translation suivi d'un mouvement de rotation de la trappe, une insertion et un guidage respectivement des ergots dans les rainures, lorsque la trappe est insérée sur le capot inférieur selon un sens d'insertion de la trappe,
- au moyen une deuxième combinaison de mouvements comprenant un mouvement de rotation suivi d'un mouvement de translation de la trappe, un guidage des ergots dans les rainures et un retrait des ergots des rainures respectivement, lorsque la trappe est retirée du capot selon un sens de retrait de la trappe opposé au sens d'insertion.
et **en ce que** :
- les rainures de chaque face sont chacune inclinée par rapport audit profil curviligne d'un angle d'inclinaison qui est croissant dans le sens de retrait de la trappe et décroissant dans le sens d'insertion de la trappe,
- les ergots de chaque face sont chacun incliné par rapport audit profil curviligne d'un angle d'inclinaison qui est croissant dans le sens de retrait de la trappe et décroissant dans le sens d'insertion de la trappe.

2. Boitier de terminal selon la revendication 1, dans lequel chaque rainure de la pluralité comprend :
- une portion d'insertion permettant une insertion en translation de l'ergot, associée à ladite rainure, dans ladite rainure, et
- une portion de guidage permettant un guidage en rotation dudit ergot dans ladite rainure.

3. Boitier de terminal selon la revendication 3, dans lequel chaque rainure se présente sensiblement sous la forme d'un L allongé longitudinalement, avec une partie latérale et une partie longitudinale, ladite partie latérale formant la portion d'insertion et ladite partie longitudinale formant la portion de guidage.

4. Boitier de terminal selon l'une quelconque des revendications 1 à 3, dans lequel chaque ergot se présente sous la forme d'un parallélépipède.

5. Boitier de terminal selon l'une quelconque des revendications 1 à 4, dans lequel :
- les rainures sont placées sur des faces latérales de la trappe, ou du capot inférieur,
- les ergots sont placés sur des faces latérales du capot inférieur, ou de la trappe respectivement.

6. Boitier de terminal selon la revendication 5, dans lequel les rainures sont réparties avec des espacements homogènes sur les faces latérales de la trappe, ou du capot inférieur, et les ergots sont répartis avec des espacements homogènes correspondants sur les faces latérales du capot inférieur, ou de la trappe respectivement.

7. Boitier de terminal selon l'une quelconque des revendications 1 à 6, comprenant des moyens de verrouillage réversibles de la trappe au capot inférieur, constitué d'au moins une patte d'accrochage (56) élastiquement flexible et orientée longitudinalement sur une extrémité avant de la trappe, coopérant avec au moins une encoche de réception de ladite au moins une patte d'accrochage, ménagée dans le capot inférieur.

8. Boitier de terminal selon l'une quelconque des revendications 1 à 6, dans lequel la trappe comprend un bouton poussoir mobile en translation, coopérant avec au moins une patte d'accrochage mobile en translation, ladite au moins une patte d'accrochage étant orientée longitudinalement sur une extrémité avant de la trappe, coopérant avec au moins une encoche de réception de ladite au moins une patte d'accrochage, ménagée dans le capot inférieur.

9. Boitier de terminal selon l'une quelconque des revendications 1 à 8, dans lequel la trappe comprend une portion (54) en forme de anse au niveau de son extrémité arrière conformée de façon à assurer un verrouillage d'au moins un connecteur situé à l'arrière du capot inférieur.

## Patentansprüche

1. Klemmenkasten (10), der mindestens eine obere Haube (30) und eine untere Haube (20) umfasst, in der eine Aufnahme (40) eingerichtet ist, die mittels einer abnehmbaren Klappe (50), die ein gekrümmtes Profil aufweist, verschlossen werden kann, **dadurch gekennzeichnet, dass**
- die Klappe oder die untere Haube eine Vielzahl von Rillen (551-558) umfasst, die längs auf mindestens zwei parallelen Seiten entlang des gekrümmten Profils verteilt sind,
- die untere Haube oder Klappe jeweils eine Vielzahl von Dornen (251-258) umfasst, die längs auf mindestens zwei parallelen Seiten entlang des gekrümmten Profils verteilt sind, wobei jeder Dorn dazu bestimmt ist, mit einer dazu gehörenden Rille zusammenzuwirken,
dass die Dorne und die Rillen ausgestaltet sind, um Folgendes zu erlauben:
- mittels einer ersten Kombination von Bewegungen, die eine Verschiebungsbewegung gefolgt von einer Drehbewegung der Klappe umfassen, ein jeweiliges Einführen und Führen der Dorne in die Rillen, wenn die Klappe auf der unteren Haube gemäß einer Einfügerichtung der Klappe eingefügt wird,
- mittels einer zweiten Kombination von Bewegungen, die eine Drehbewegung gefolgt von einer Verschiebungsbewegung der Klappe umfassen, jeweils ein Führen der Dorne in den Rillen und ein Zurückziehen der Dorne aus den Rillen, wenn die Klappe von der Haube entlang einer Rückzugrichtung der Klappe, die der Einfügerichtung entgegengesetzt ist, entfernt wird,
und dass:
- die Rillen jeder Seite jeweils in Bezug zu dem gekrümmten Profil um einen Neigungswinkel geneigt sind, der in die Rückzugrichtung der Klappe zunimmt und in die Einfügerichtung der Klappe abnimmt,
- die Dorne jeder Seite jeweils in Bezug zu dem gekrümmten Profil um einen Neigungswinkel geneigt sind, der in die Rückzugrichtung der Klappe zunimmt und in die Einfügerichtung der Klappe abnimmt.

2. Klemmenkasten nach Anspruch 1, wobei jede Rille der Vielzahl Folgendes umfasst:
- einen Einfügeabschnitt, der ein Einfügen in Verschiebung des Dorns verbunden mit der Rille, in die Rille erlaubt, und
- einen Führungsabschnitt, der ein Führen in Drehung des Dorns in der Rille erlaubt.

3. Klemmenkasten nach Anspruch 3, wobei jede Rille im Wesentlichen die Form eines längs gestreckten L aufweist, mit einem seitlichen Teil und einem Längsteil, wobei der seitliche Teil den Einfügeabschnitt bildet und der Längsteil den Führungsabschnitt bildet.

4. Klemmenkasten nach einem der Ansprüche 1 bis 3, wobei jeder Dorn die Form eines Parallelepipeds aufweist.

5. Klemmenkasten nach einem der Ansprüche 1 bis 4, wobei:
- die Rillen auf seitlichen Seiten der Klappe oder der unteren Haube positioniert sind,
- die Dorne jeweils auf seitlichen Seiten der unteren Haube oder auf der Klappe platziert sind.

6. Klemmenkasten nach Anspruch 5, wobei die Rillen mit homogenen Beabstandungen auf den seitlichen Seiten der Klappe oder der unteren Haube verteilt sind, und die Dorne mit entsprechenden homogenen Beabstandungen jeweils auf den seitlichen Seiten der unteren Haube oder der Klappe verteilt sind.

7. Klemmenkasten nach einem der Ansprüche 1 bis 6, der umkehrbare Mittel zum Verriegeln der Klappe an der unteren Haube umfasst, die mindestens aus einer Anhängpratze (56), die elastisch biegsam und längs auf einem vorderen Ende der Klappe ausgerichtet ist, besteht, die mit mindestens einer Aufnahmekerbe der mindestens einen Anhängpratze, die in der unteren Haube eingerichtet ist, zusammenwirkt.

8. Klemmenkasten nach einem der Ansprüche 1 bis 6, wobei die Klappe einen Druckknopf umfasst, der in Verschiebung beweglich ist, der mit mindestens einer in Verschiebung beweglichen Anhängpratze zusammenwirkt, wobei die mindestens eine Anhängpratze auf einem vorderen Ende der Klappe längs ausgerichtet ist, in dem sie mit mindestens einer Aufnahmekerbe der mindestens einen Anhängpratze, die in der unteren Haube eingerichtet ist, zusammenwirkt.

9. Klemmenkasten nach einem der Ansprüche 1 bis 8, wobei die Klappe einen Abschnitt (54) in Henkelform im Bereich ihres hinteren Endes umfasst, der derart ausgebildet ist, dass er ein Verriegeln mindestens eines Steckverbinders, der sich auf der Rückseite der unteren Haube befindet, sicherstellt.

## Claims

1. Terminal casing (10) comprising at least one upper cover (30) and one lower cover (20) in which there is made a housing (40) that can be closed by means of a removable hatch (50) having a curvilinear profile, **characterized in that**:
- the hatch, or the lower cover, comprises a plurality of grooves (55₁-55₈) distributed longitudinally on at least two parallel faces in following said curvilinear profile,
- the lower cover, or the hatch respectively, comprises a plurality of tabs (25₁-25₈) distributed longitudinally on at least two parallel faces in following said curvilinear profile, each tab being designed to cooperate with an associated groove,
**in that** the tabs and the grooves are shaped so as to enable:
- through a first combination of motions comprising a translational motion followed by a rotational motion of the hatch, an insertion and a guidance respectively of the tabs in the grooves, when the hatch is inserted into the lower cover according to a direction of insertion of the hatch,
- through a second combination of motions comprising a rotational motion followed by a translational motion of the hatch, a guidance of the tabs in the grooves and a withdrawal of the tabs from the grooves respectively, when the hatch is withdrawn from the cover according to a direction of withdrawal of the hatch opposite to the direction of insertion;
and **in that**:
- the grooves of each face are each tilted relative to the curvilinear profile by an angle of tilt that increases in the direction of withdrawal of the hatch and decreases in the direction of insertion of the hatch,
- the tabs of each face are each tilted relative to the curvilinear profile by an angle of tilt that increases in the direction of withdrawal of the hatch and decreases in the direction of insertion of the hatch.

2. Terminal casing according to claim 1, wherein each groove of the plurality comprises:
- an insertion portion enabling an insertion in translation of the tab associated with said groove, in said groove, and
- a guiding portion enabling a guidance in rotation of said tab in said groove.

3. Terminal casing according to claim 3, wherein each groove substantially takes the shape of an L reclining longitudinally, with a lateral part and a longitudinal part, said lateral part forming the insertion portion and said longitudinal part forming the guiding portion.

4. Terminal casing according to any one of the claims 1 to 3, wherein each tab takes the shape of a parallelepiped.

5. Terminal casing according to any one of the claims 1 to 4, wherein:
- the grooves are placed on lateral faces of the hatch, or the lower cover,
- the tabs are placed on lateral faces of the lower cover, or the hatch respectively.

6. Terminal casing according to claim 5, wherein the grooves are distributed with homogenous spacing on the lateral faces of the hatch, or the lower cover, and the tabs are distributed with homogenous spacing on the lateral faces of the cover, or the hatch respectively.

7. Terminal casing according to any one of the claims 1 to 6, comprising means for reversibly locking the hatch to the lower cover, constituted by at least one elastically flexible, longitudinally oriented coupling toe (56) on one front end of the hatch, cooperating with at least one notch for receiving said at least one coupling toe, made in the lower cover.

8. Terminal casing according to any one of the claims 1 to 6, wherein the hatch comprises a push-button mobile in translation, cooperating with at least one coupling toe mobile in translation, said at least one coupling toe being oriented longitudinally on a front end of the hatch, cooperating with at least one reception notch of said at least one coupling toe made in the lower cover.

9. Terminal casing according to any one of the claims 1 to 8, wherein the hatch comprises a bow-shaped portion (54) at its rear end shaped so as to ensure a locking of at least one connector situated behind the lower cover.
